# EUROPEAN PATENT APPLICATION

(11) **EP 3 308 784 A1**
(43) Date of publication of application: **18.04.2018**
(21) Application number: 16807577.8
(22) Date of filing: 09.06.2016
(51) Int. Cl.: A61K 31/427, A61P 27/02

(54) **THERAPEUTIC AGENT FOR NEUROTROPHIC KERATOPATHY**

(30) Priority: 09.06.2015 JP 2015116974
(71) Applicant: Senju Pharmaceutical Co., Ltd., Osaka-shi, Osaka 541-0046 (JP)
(72) Inventor: KOBAYASHI, Shinya, Osaka-shi Osaka 541-0046 (JP); NAKAMURA, Yoshikuni, Osaka-shi Osaka 541-0046 (JP); TARUI, Takeshi, Osaka-shi Osaka 541-0046 (JP); WADA, Tomoyuki, Osaka-shi Osaka 541-0046 (JP)
(74) Representative: Webster, Jeremy Mark
(86) International application number: PCT/JP2016/067288
(87) International publication number: WO 2016/199871

(57) **Abstract**

Provided are a novel pharmaceutical use of PPARδ agonist such as a therapeutic agent for neurotrophic keratopathy, an improving agent for decrease of corneal sensitivity and the like, each containing [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof.

## Description

### [Technical Field]

The present invention relates to a therapeutic drug for neurotrophic keratopathy, and a novel pharmaceutical use of PPAR (Peroxisome Proliferator-Activated Receptor) δ agonist as a therapeutic drug for neurotrophic keratopathy.

### [Background Art]

PPAR is one type of nuclear receptor expressed in most vertebrates, and is considered to be a transcription factor group closely involved in intracellular sugar/lipid metabolism and cell differentiation. As subtypes, α, δ, γ types are known. PPARδ is sometimes indicated as PPARβ (non-patent document 1).

As the distribution of PPAR in eye tissues, expression of PPARα and β in corneal epithelial cells of rabbits is known (non-patent document 2).

It has heretofore been reported that 5-[4-(6-methoxy-1-methyl-1H-benzimidazol-2-ylmethoxy)benzyl]thiazolidine-2,4-dione considered to mainly have a PPARγ activating action can be utilized as a therapeutic agent for keratoconjunctival disorders (patent documents 1 and 2), and PPARα, δ or γ agonists are administered for the treatment of ocular diseases (conjunctivitis, dry eye syndrome, keratitis etc.) (patent document 3). PPARα is known to be distributed in the liver, kidney and the like and act on lipid metabolism/transportation and further, it has also been reported that PPARα agonist can be utilized as a therapeutic agent for corneal diseases (patent document 4). PPARδ agonist has heretofore been reported to promote proliferation and differentiation of rat sebaceous gland epithelial cells (non-patent document 3) and promote skin wound healing (non-patent document 4). Besides these, a method of stimulating proliferation of β cells by administering non-thiazolidinedione PPAR ligand and GLP-1 derivative (patent document 5), inhibition of proliferation of leukemia cells, prostate cancer cells and the like by pioglitazone, a PPARγ agonist (patent document 6) and the like are known.

The present inventors have found that a particular PPARδ agonist has a promoting action on the proliferation of corneal epithelial cells and is useful for the treatment of corneal epithelial disorders and corneal epithelial disorders associated with various diseases: corneal epithelial disorders associated with endogenous diseases such as Sjogren's syndrome, Stevens-Johnson syndrome, karatoconjuctivitis sicca (dry eye) and the like; corneal epithelial disorders associated with exogenous diseases in post-operation, drug use, trauma, corneal ulcer, meibomitis, contact lens wearing or the like; and corneal epithelial disorders associated with ocular allergic diseases with corneal lesions such as vernal keratokonjuncitivitis, atopic keratoconjunctivitis and the like (patent document 7). However, patent document 7 does not suggest that PPARδ agonist shows an effect to improve decrease of corneal sensitivity and thus can be utilized as a therapeutic agent for neurotrophic keratopathy.

### [Document List]

### [Patent documents]

patent document 1: JP-B-4216266
patent document 2: JP-A-2006-304611
patent document 3: JP-B-3756313
patent document 4: JP-B-4731324
patent document 5: JP-B-4383178
patent document 6: JP-B-5030998
patent document 7: JP-B-5247686 (WO 2008/143254)

### [non-patent document]

non-patent document 1: J Med Chem 2000, 43: 527-550
non-patent document 2: J Biol Chem 2000, 275: 2837-2844
non-patent document 3: Molecular Genetic and Metabolism 2001, 74: 362-369
non-patent document 4: Am J Clin Dermatol 2003, 4(8): 523-530

### [SUMMARY OF THE INVENTION]

### [Problems to be Solved by the Invention]

The problem to be solved by the present invention is provision of a new pharmaceutical use of PPARδ agonist in the ophthalmologic field.

### [Means of Solving the Problems]

In view of the above-mentioned problems, the present inventors have conducted intensive studies and found that a particular PPARδ agonist is superior in the treatment effect on neurotrophic keratopathy, which resulted in the completion of the present invention.

Therefore, the present invention includes at least the following.
[1] A therapeutic agent for neurotrophic keratopathy comprising [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof.
[2] The therapeutic agent of [1], wherein the neurotrophic keratopathy is neurotrophic keratopathy caused by diabetes.
[3] The therapeutic agent of [2], wherein the neurotrophic keratopathy caused by diabetes is diabetic keratopathy.
[3-1] The therapeutic agent of [1] - [3] for topical ophthalmic administration.
[4] An agent for improving decrease of corneal sensitivity, comprising [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof.
[4-1] The improving agent of [4] for topical ophthalmic administration.
[5] Use of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof for the manufacture of a therapeutic agent for neurotrophic keratopathy.
[6] The use of [5], wherein the neurotrophic keratopathy is neurotrophic keratopathy caused by diabetes.
[7] The use of [6], wherein the neurotrophic keratopathy caused by diabetes is diabetic keratopathy.
[8] Use of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof for the manufacture of an improving agent for decrease of corneal sensitivity.
[9] A method of treating neurotrophic keratopathy, comprising administering an effective amount of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof to a subject in need of a treatment of neurotrophic keratopathy.
[10] The method of [9], wherein the neurotrophic keratopathy is neurotrophic keratopathy caused by diabetes.
[11] The method of [10], wherein the neurotrophic keratopathy caused by diabetes is diabetic keratopathy.
[12] A method of improving decrease of corneal sensitivity, comprising administering an effective amount of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof to a subject in need of an improvement of decrease of corneal sensitivity.
[13] A therapeutic agent for corneal neuropathy, comprising [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof.
[13-1] The therapeutic agent of [13], wherein the corneal neuropathy is a disorder due to diabetes.
[13-2] The therapeutic agent of [13] or [13-1] for topical ophthalmic administration.

### [Effect of the Invention]

According to the present invention, a therapeutic agent for neurotrophic keratopathy is provided. Neurotrophic keratopathy is a corneal disease developed when corneal nerves, which control corneal sensitivity, are disordered by being damaged, degenerated or paralyzed. The clinical findings thereof include decrease of corneal sensitivity, disorder of corneal epithelial cells, and delay in wound healing. In the treatment of this disease, seeking the cause in the cornea itself or treatment of the corneal epithelial disorder itself often results in a failure to sufficiently achieve the object. However, since the active ingredient of the present invention has an improving effect on the decrease of corneal sensitivity, it is extremely useful as a therapeutic agent for neurotrophic keratopathy. Neurotrophic keratopathy and decrease of corneal sensitivity are caused by herpes keratitis, surgical trauma and the like, as well as keratopathy as a complication of diabetes. Especially, since not only proliferation of corneal epithelial cells but also improvement of decrease of corneal sensitivity are important for the treatment of diabetic keratopathy, the medicament of the present invention is useful as a therapeutic agent for diabetic keratopathy.

### [Brief Description of the Drawings]

Fig. 1 shows an outline of production of a flap of rabbit cornea and the measurement locations (1-4) of corneal sensitivity.
Fig. 2 is a graph of corneal sensitivity at measurement location 1 after production of a corneal flap.
Fig. 3 is a graph of corneal sensitivity at measurement location 2 after production of a corneal flap.

### [Description of Embodiments]

The present invention provides a therapeutic agent for neurotrophic keratopathy, an improving agent for decrease of corneal sensitivity, a therapeutic agent for diabetic keratopathy, and a therapeutic agent for corneal neuropathy, each containing [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof (hereinafter to be also referred to generically as "the compound of the present invention") as an active ingredient. The therapeutic agent for neurotrophic keratopathy, improving agent for decrease of corneal sensitivity, and therapeutic agents for diabetic keratopathy and corneal neuropathy of the present invention are generically referred to as the medicament of the present invention.

The "neurotrophic keratopathy" in the present invention is a corneal disease developed when corneal nerves, which control corneal sensitivity, are disordered by being damaged, degenerated or paralyzed. The clinical findings thereof include decrease of corneal sensitivity, disorder of corneal epithelial cells, and delay in wound healing.

The "decrease of corneal sensitivity" in the present invention refers to a state in which corneal nerves are damaged, degenerated or paralyzed to show decline in the sensitivity of the corneal nerves.

The "corneal nerve" in the present invention is under control of the trigeminal nerve, which is a sensory nerve, and refers to limbal plexus formed around the periphery of cornea, stroma plexus distributed in a mesh pattern in the corneal stroma, sub-epithelial nerve plexus formed directly under the Bowman's membrane, sub-basal nerve plexus and nerve fiber formed at the point of penetration of the Bowman's membrane.

The "treatment" in the present invention is a concept including not only improvement of symptoms, delay of progression and prevention of severity, but also prophylaxis. The prophylaxis means suppression or delaying of the onset of symptoms, and means, in detail, suppression or delaying of the onset of clinical findings by recovering the disorder of corneal nerves in "neurotrophic keratopathy", which is developed when corneal nerves are disordered by being damaged, degenerated or paralyzed due to various reasons and which shows decrease of corneal sensitivity, disorder of corneal epithelial cells, and delay in wound healing as the clinical findings.

Specifically, the prophylaxis of "neurotrophic keratopathy" means suppression or delaying of the onset of the above-mentioned clinical findings by administering, before exteriorization of the clinical findings, the compound of the present application to subjects having various etiologies.

Examples of the various etiologies include diabetes, aging, cornea transplantation, eye surgery, contact lens wearing, corneal herpes, trigeminal nerve paralysis and the like.

The degree of corneal epithelial disorder can be confirmed by observing fluorescein-stained corneal epithelium with a slit lamp, and using AD Classification (Japanese Journal of Clinical Ophthalmology 1994, 48, 183-188).

The degree of decrease of corneal sensitivity can be confirmed by measuring the sensitivity at a particular measurement point on the corneal surface by using Cochet-Bonnet corneal esthesiometer (manufactured by LUNEAU). For example, accurate definition of normal values for human is not possible since the corneal sensitivity decreases by aging. It is important to define the measurement points and compare the left and right eyes.

The compound of the present invention provides an effect of promoting the improvement of decrease of corneal sensitivity and therefore, is useful for the treatment of neurotrophic keratopathy. Diabetes is known to cause nerval disorders, and neuropathy is known to further cause corneal epithelial disorders. Thus, the compound of the present invention is particularly useful for the treatment of neurotrophic keratopathy caused by diabetes and further, diabetic keratopathy that accompanies damage, degeneration or paralysis of corneal nerve. In addition, the compound of the present invention has a recovery effect on damaged nerves and therefore, is useful for preventing corneal epithelial disorders caused by neuropathy, particularly diabetic keratopathy.

The compound of the present invention has a recovery effect on the disorders of the corneal nerves and therefore, is useful for treating corneal neuropathy.

Corneal neuropathy is caused by diabetes, aging, corneal transplantation, eye surgery, contact lens wearing, corneal herpes, trigeminal nerve paralysis and the like. Among others, it is useful for treating corneal neuropathy caused by diabetes.

### 3-[2-[4-Isopropyl-2-(4-trifluoromethyl)phenyl-5-thiazolyl]ethyl]-5-methyl-1,2-benzisoxazol-6-yl]oxyacetic acid (CAS No.515138-06-4):

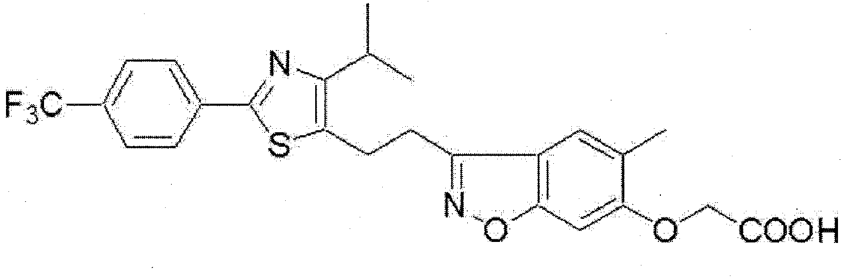

, which is contained as an active ingredient in the medicament of the present invention, is a compound having a PPARδ agonist activity and described in WO 2003/033493 (particularly Example 5) .

Examples of a pharmacologically acceptable salt of the above-mentioned compound include metal salts with alkali metals such as sodium, potassium etc.; alkaline earth metals such as calcium, magnesium etc., and the like. The compound of the present invention also includes solvates thereof.

The PPARδ agonist in the present invention refers to a substance that binds to the ligand binding domain (LBD) of PPARδ, activates the receptor and modulates transcription of the PPAR target gene. To remove influence of other intrinsic nuclear receptors in mammalian cells, the PPARδ agonist activity may be measured by a yeast two-hybrid method using a chimeric receptor of LBD and yeast GAL4, and a reporter gene. Specific measurement methods include PPAR-GAL4 assays described in reference literatures, T.M. Willson et al., Journal of Medicinal Chemistry, 2000, vol.43, No.4, p.528-550 and J.M. Lehmann et al., The Journal of Biological Chemistry, 1995, vol.270, No.22, p.12953-12956. The compound of the present invention has been confirmed to have a PPARδ agonist activity by the method described in Example 12 of WO 2003/033493.

The compound of the present invention can be synthesized according to the description in WO 2003/033493 (particularly Example 5).

The medicament of the present invention can contain any carrier in addition to the above-mentioned active ingredient. Examples of such carrier include solvents (e.g., water, alcohol etc.), buffering agents (e.g., phosphate buffer, acetate buffer, borate buffer, carbonate buffer, citrate buffer, tris buffer, glutamic acid, epsilon aminocaproic acid etc.), preservatives (e.g., benzalkonium chloride, benzethonium chloride, chlorhexidine gluconate, chlorobutanol, benzyl alcohol, sodium dehydroacetate, paraoxybenzoates, sodium edetate, boric acid etc.), isotonicity agents (e.g., sodium chloride, potassium chloride, glycerol, mannitol, sorbitol, boric acid, glucose, propylene glycol etc.) and the like.

Examples of the subject of administration of the medicament of the present invention include mammals (e.g., human, mouse, rat, hamster, rabbit, cat, dog, bovine, sheep, monkey etc.).

The medicament of the present invention is used in a dosage form such as eye drop, adhesive preparation, ointment, lotion, cream, oral preparation or the like, and can contain any carrier, for example, pharmaceutically acceptable carrier in addition to the above-mentioned active ingredient.

While the administration route of the medicament of the present invention is not particularly limited as long as the aforementioned treatment effects are afforded, it is preferably topical ophthalmic administration. A dosage form for topical ophthalmic administration is, for example, eye drop or ophthalmic ointment.

For example, when the medicament of the present invention is used as an eye drop or ophthalmic ointment, stabilizers (e.g., sodium bisulfite, sodium thiosulfate, sodium edetate, sodium citrate, ascorbic acid, dibutylhydroxytoluene etc.), solubilizing agents (e.g., glycerol, propylene glycol, macrogol, polyoxyethylene hydrogenated castor oil etc.), suspending agents (e.g., polyvinylpyrrolidone, hydroxypropylmethylcellulose, hydroxymethylcellulose, sodium carboxymethylcellulose etc.), emulsifiers (e.g., polyvinylpyrrolidone, soybean lecithin, egg-yolk lecithin, polyoxyethylene hydrogenated castor oil, polysorbate 80 etc.), buffering agents (e.g., phosphate buffer, acetate buffer, borate buffer, carbonate buffer, citrate buffer, tris buffer, glutamic acid, epsilon aminocaproic acid etc.), thickening agents (e.g., water-soluble cellulose derivatives such as methylcellulose, hydroxyethylcellulose, hydroxypropylmethylcellulose, carboxymethylcellulose and the like, sodium chondroitin sulfate, sodium hyaluronate, carboxyvinyl polymer, poly(vinyl alcohol), polyvinylpyrrolidone, macrogol etc.), preservatives (e.g., benzalkonium chloride, benzethonium chloride, chlorhexidine gluconate, chlorobutanol, benzyl alcohol, sodium dehydroacetate, paraoxybenzoates, sodium edetate, boric acid etc.), isotonicity agents (e.g., sodium chloride, potassium chloride, glycerol, mannitol, sorbitol, boric acid, glucose, propylene glycol etc.), pH adjusters (e.g., hydrochloric acid, sodium hydroxide, phosphoric acid, acetic acid etc.), algefacients (e.g., 1-menthol, d-camphor, d-borneol, peppermint oil etc.), ointment bases (e.g., white petrolatum, purified lanolin, liquid paraffin, vegetable oils (olive oil, camellia oil, peanuts oil etc.) etc.) and the like can be added as additive. While the amount of these additives to be added varies depending on the kind, use and the like of the additive, the additives may be added to a concentration capable of achieving the object of the additive.

When the medicament of the present invention is formed as an eye drop or ophthalmic ointment, it may be produced according to a method generally used in the pharmaceutical field. For example, it can be produced based on the methods described in the sections of eye drop and ophthalmic ointment in the Japanese Pharmacopoeia, the 16th Edition, Preparation General Rules.

Examples of the form of the eye drop include aqueous eye drops (aqueous ophthalmic solution, aqueous ophthalmic suspension, viscous ophthalmic solution etc.), non-aqueous eye drops (non-aqueous ophthalmic solution, non-aqueous suspension etc.), emulsion eye drop and the like.

While the pH of eye drop is appropriately determined according to the form of the eye drop, it is generally within the range of 4 - 8. When the eye drop is an aqueous ophthalmic solution, the pH is particularly preferably set to 6 - 8 from the aspect of the solubility of the active ingredient.

The eye drop is generally a preparation sterilized by a method such as sterilization by filtration, radiation sterilization (e.g., electron beam sterilization, UV sterilization, gamma sterilization etc.), autoclave sterilization, dry heat sterilization and the like.

When formulated as an eye drop, the liquid is preferably stored in an eye drop container provided with a liquid injection hole with a small diameter, which is capable of controlling the droplet quantity to facilitate dripping to the eye. As the material of the container, synthetic resin, glass, cellulose, pulp and the like are used, and the material is appropriately selected according to the properties and amount of use of the active ingredient and the base materials. From the aspects of squeezability and durability, the container is preferably made of a synthetic resin. Specific materials of the synthetic resin include, for example, polyethylene resin (e.g., low density polyethylene or high density polyethylene), polypropylene resin, ethylene-propylene copolymer resin, poly(ethylene terephthalate)resin and the like.

Examples of the eye drop container include a container in which a container body and an inner plug member molded separately are fitted together, an integrally-molded filling container in which a liquid is filled with sealing simultaneously with molding of the container (e.g., WO 2004/006826) and the like. The integrally-molded filling container is superior in terms of cost or hygiene, since the container and the liquid are integrally produced. The eye drop container may be a unit dose type container for one-time use only (e.g., JP-A-9-207959). Using this container, a preparation highly safe for the cornea and free of preservative can be formed. These containers may be tightly packaged with a UV shielding film. The container may be colored (brown, green, blue, yellow etc.) to enhance the UV shielding performance.

The present invention provides a method of treating neurotrophic keratopathy or diabetic keratopathy, comprising administering an effective amount of the compound of the present invention to a subject in need of a treatment of neurotrophic keratopathy or diabetic keratopathy.

Similarly, the present invention provides a method of preventing neurotrophic keratopathy or diabetic keratopathy, comprising administering an effective amount of the compound of the present invention to a subject at risk of being affected with or expected to be affected with neurotrophic keratopathy or diabetic keratopathy. The aforementioned subject includes, for example, a subject affected with diabetes, a subject having a risk factor of corneal herpes, contact lens wearing, eye surgery, aging and the like.

Diabetic keratopathy is also a disease associated with decrease of corneal sensitivity. Therefore, the present invention provides a method of improving decrease of corneal sensitivity, comprising administering an effective amount of the compound of the present invention to a subject in need of an improvement of decrease of corneal sensitivity.

The present invention provides a method of preventing decrease of corneal sensitivity, comprising administering an effective amount of the compound of the present invention to a subject at risk of or expected to have decrease of corneal sensitivity. The aforementioned subject includes, for example, a subject affected with diabetes.

The present invention provides a method of treating corneal neuropathy, comprising administering an effective amount of the compound of the present invention to a subject in need of a treatment of corneal neuropathy.

The effective amount of the compound of the present invention cannot be automatically defined irrespective of the age, body weight, condition, treatment object and the like of the subject of administration. When the medicament of the present invention is administered to human, for example, the effective amount is an amount of 1 - 2 drops for one eye per one time, which is given 3-4 times per one day, of a solution containing the compound of the present invention at generally 0.0001 - 0.1 w/v%, preferably 0.001 - 0.01 w/v%, most preferably 0.005 w/v%, that is, about 10 - 100 µL per one time. The amount of the compound in the solution at such concentration and volume can be recited as an example of the effective amount.

### [Examples]

While the present invention is explained in detail in the following by referring to Experimental Examples, the present invention is not limited thereby in any manner. In the Experimental Examples, % means w/v% unless particularly indicated.

### (Experimental Example 1)

### Effect of compound A on decrease of corneal sensitivity of rabbit

### 1. Animals used

Male Japanese white rabbits (body weight 2.80 - 3.32 kg) purchased from KITAYAMA LABES Co., Ltd. were used. The animals were bred in a breeding room set to room temperature 23°C ± 3°C, humidity 55% ± 10% RH, 12 hr lighting (08:00 lighting, 20:00 lights-out) from the arrival to the test end date. A solid feed (Labo R Stock, Nosan Corporation, 100 - 120 g/day) was given to the animals, and the animals were allowed to freely ingest tap water, passed through a sterilizer for animal drink, from an automatic-water supply apparatus. The test was performed on an approval from the animal care and use committee based on the Act on Welfare and Management of Animals (Act No. 105 of October 1, 1973, final revision: Act No. 38 of June 12, 2013) and the like.

### 2. Test substances

As a test substance, [3-[2-[4-isopropyl-2-(4-trifluoromethyl)phenyl-5-thiazolyl]ethyl]-5-methyl-1,2-benzisoxazol-6-yl]oxyacetic acid (hereinafter to be referred to as compound A) was used. Compound A was dissolved in the vehicle shown in Table 1 to 0.005% and used as an ophthalmic solution. As a control group, a vehicle ophthalmic solution made solely of a vehicle (Table 1) and free of compound A was used. The label of the ophthalmic solution was encrypted so that the corneal sensitivity measurer would not know which ophthalmic solution was administered to the rabbit under measurement (single blind test).

**[Table 1]**

| component | content (in 100 mL of formulation) |
|---|---|
| sodium dihydrogen phosphate | 0.1 g |
| concentrated glycerol | 2.4 g |
| 1% Tyloxapol solution | 10 mL |
| 1 mol/L sodium hydroxide test solution | q.s. |
| purified water | q.s. |
| total amount | 100 mL (pH 7.5) |

### 3. Test method 1) Assignment

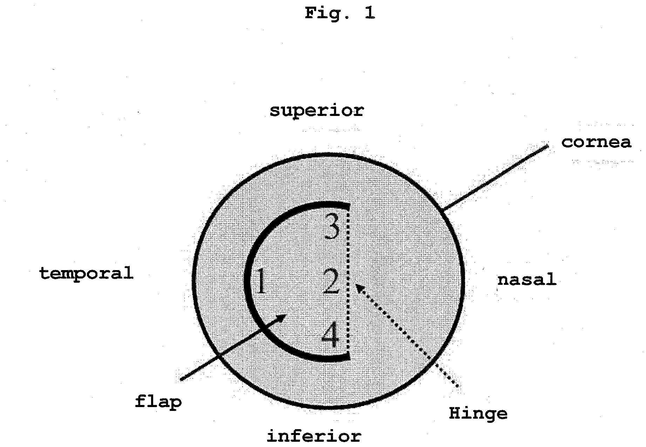

Before production of corneal flap, the anterior ocular segment of the rabbits was macroscopically observed, and the rabbits showing no abnormality were selected. Then, using Cochet-Bonnet corneal esthesiometer (manufactured by LUNEAU), the corneal sensitivity at measurement locations 1 and 2 in Fig. 1 was measured, and the rabbits showing a corneal sensitivity threshold of not less than 15 mm at both measurement locations 1 and 2 were selected. The selected 16 rabbits were assigned to two groups of compound A ophthalmic solution administration group and vehicle ophthalmic solution administration group (8 for each group) such that the corneal sensitivity threshold of each group was uniform.

### 2) Production of corneal flap

The rabbits generally anesthetized by intramuscular injection of 1:2 mixture (0.9 mL/kg) of xylazine hydrochloride injection (Celactal 2% injection (registered trade mark), Bayer, Ltd.) and ketamine hydrochloride injection (Ketalar intramuscular injection 500 mg (registered trade mark), DAIICHI SANKYO COMPANY, LIMITED), and topically anesthetized using 0.4% oxybuprocaine hydrochloride ophthalmic solution (Benoxil ophthalmic solution 0.4% (registered trade mark), Santen Pharmaceutical Co., Ltd.). Eyeballs were sufficiently exposed, and a corneal flap (thickness 130 µm, diameter 8.6 mm) was produced using microkeratome (MK-2000, manufactured by NIDEK CO., LTD.). After reattachment of the flap under a surgical microscope, the rabbits were laid with the site of operation up until awakening from the anesthesia. After awakening, 0.3% gatifloxacin ophthalmic solution (Gatiflo ophthalmic solution (registered trade mark), Senju Pharmaceutical Co., Ltd.) was administered by instillation.

### 3) Administration

To the rabbits free of flap detachment and infection of cornea, and free of sensitivity at any of the measurement locations 1 - 4 (Fig. 1) on the next day of flap production was administered by instillation of compound A ophthalmic solution or vehicle ophthalmic solution for 17 days. The instillation administration was performed by instillation of 50 µL/administration 4 times per day (twice per day on the next day of operation and 14, 16 and 21 days after operation, and 3 times per day at 4 days after operation) at intervals of not less than 2 hr to each treated eye with a micropipette. At 1, 2, 4, 5 and 6 days from the operation, 0.3% gatifloxacin ophthalmic solution was administered by instillation to the treated eyes not less than 10 minutes before instillation administration of the compound A ophthalmic solution or vehicle ophthalmic solution.

### 4) Corneal sensitivity measurement

The corneal sensitivity was measured using Cochet-Bonnet corneal esthesiometer at measurement locations 1 and 2 (Fig. 1) on the next day of flap production and 1, 2, 3 and 4 weeks after the production.

### 4. Test results

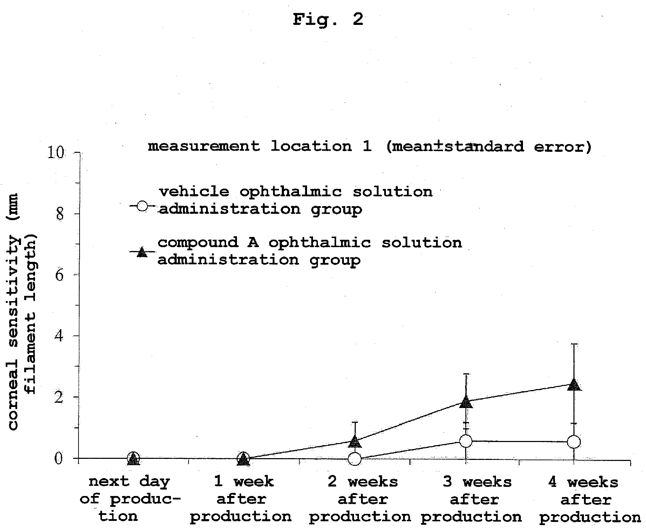

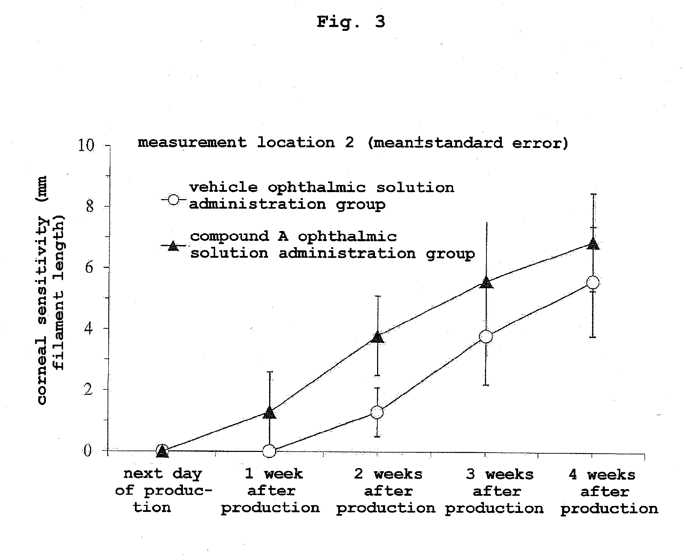

The test results are shown in Fig. 2 and Fig. 3. The sensitivity was recovered faster in the compound A ophthalmic solution administration group than in the vehicle ophthalmic solution administration group. As demonstrated above, it was clarified that compound A promotes recovery of the nerves damaged by flap production and causes early recovery of decrease of corneal sensitivity associated with neuropathy.

### (Experimental Example 2)

### Effect of compound A on corneal epithelial disorder in spontaneously diabetic rat (Goto-Kakizaki (GK) rat)

### 1. Animals used

Male GK rats (20 rats) (Wakuta M., et al., Invest. Ophthalmol. Vis. Sci. 2007 48 590-596) purchased from CLEA Japan, Inc. were used. Corneal epithelial disorders associated with neuropathy were found in GK rats (Wang F., et al., Am J Pathol. 2012 181 2058-2066). GK rats are originated from Wistar rats and therefore, Wistar rats were used as normal control group (7 rats). The animals were bred in a breeding room set to room temperature 23°C ± 3°C, humidity 55% ± 10% RH, 12 hr lighting (08:00 lighting, 20:00 lights-out) from the arrival to the test end date. The animals were allowed to freely ingest a solid feed (Labo MR Stock, Nosan Corporation) and freely ingest tap water, passed through a sterilizer for animal drink, from an automatic-water supply apparatus. The test was performed on an approval from the animal care and use committee based on the Act on Welfare and Management of Animals (Act No. 105 of October 1, 1973, final revision: Act No. 38 of June 12, 2013) and the like.

### 2. Test substance

As a test substance, compound A was used. Compound A was dissolved in the vehicle (Table 2) to 0.005% and used as an ophthalmic solution. As a control group, a vehicle ophthalmic solution made solely of a vehicle (Table 2) and free of compound A was used.

**[Table 2]**

| component | content (in 100 mL of formulation) |
|---|---|
| sodium dihydrogenphosphate dihydrate | 0.1 g |
| sodium chloride | 0.9 g |
| polysorbate 80 | 0.1 mL |
| sodium hydroxide test solution | q.s. |
| purified water | q.s. |
| total amount | 100 mL (pH 7.0) |

### 3. Test method

### 1) Assignment

GK rats were assigned to three groups of compound A ophthalmic solution administration group (4 rats) and non-administration group and vehicle ophthalmic solution administration group (8 for each group) using a statistical analysis software (SAS clinical package ver. 5.0, SAS Institute Japan), such that mean blood glucose concentration and mean corneal epithelial disorder score measured before instillation administration were uniform.

### 2) Administration

Compound A ophthalmic solution or vehicle ophthalmic solution was administered by instillation for 14 days. Instillation administration was performed 4 times per day at 10 µL/administration to both eyes with a micropipette. The administration was not performed on the non-administration group and Wistar rats.

### 3) Measurement of blood glucose concentration

The blood glucose concentration was measured using glucose CII Test Wako (Wako Pure Chemical Industries, Ltd.) before the instillation administration.

### 4) Observation of corneal epithelial disorder

0.1% Sodium fluoresceinate (Wako Pure Chemical Industries, Ltd.) (5 µL) was administered by instillation and fluorescein staining of the corneal epithelium was observed using a slit lamp, whereby the degree of corneal epithelial disorder was determined. To show the degree of abnormal findings in detail, one item was added to the diagnostic criteria of the McDonald-Shadduck method and used as the diagnostic criteria of the test (Table 3). The corneal epithelial disorder was scored for each of the three (upper, middle and lower) regions and the scores of the 3 regions were added up together. The corneal epithelial disorder was observed 2, 4, 6, 8, 11 and 15 days after the instillation administration.

**[Table 3]**

| score | diagnostic criteria |
|---|---|
| 0 | no fluorescein staining |
| 1 | Slight staining plaques are observed in a small range. Observation of bottom structure by scattering light is easy. |
| 1.5 (added item) | Staining plaques of moderate degree are scattered. With scattered distribution thereof, observation of bottom structure by scattering light is easy. |
| 2 | Staining plaques of moderate degree are observed in a small range. Details are not clear in observation of bottom structure by scattering light. |
| 3 | Remarkable staining plaques are found. Staining plaques are found in wide range of cornea. Observation of bottom structure by scattering light is difficult. |
| 4 | Extreme staining plaques are found. Observation of bottom structure by scattering light is not possible. |

### 4. Test results

As shown in Table 4, a comparison of the non-administration group and the vehicle ophthalmic solution administration group has clarified that corneal epithelial disorder is improved in the GK rats of the compound A ophthalmic solution administration group. As demonstrated above, compound A is useful for the treatment of diabetic keratopathy.

**[Table 4]**

| Group | before administration | 8 days after administration | 11 days after administration | 15 days after administration |
|---|---|---|---|---|
| non-administration group | 4.59 ± 0.16 | 4.81 ± 0.12 | 5.03 ± 0.07 | 4.84 ± 0.09 |
| vehicle ophthalmic solution administration group | 4.50 ± 0.14 | 4.56 ± 0.10 | 4.53 ± 0.10 | 4.44 ± 0.09 |
| compound A ophthalmic solution administration group | 4.63 ± 0.33 | 4.06 ± 0.20 | 4.00 ± 0.13 | 4.00 ± 0.13 |
| Wistar rat | - | - | - | 3.71 ± 0.15 |

Diabetic keratopathy is a disease having diabetes as the background and involving decrease of corneal sensitivity. Substance P is considered to act as a neurotropic factor for corneal epithelial cells since the cornea i) contains substance P positive nerve fibers, ii) substance P or substance P metabolite in the tear fluid even in healthy humans, and iii) decreased substance P in the tear fluid when the corneal sensitivity is low. In addition, it has been reported that substance P as a neurotransmitter of the sensory nerve system promotes corneal epithelium wound healing in cooperation with growth factors (Teruo Nishida, Kakumaku tekisuto, page 164, published March 30, 2010, published by Elsevier Japan KK). In view of such findings, since compound A promotes secretion of neurotropic factors by improving decrease of corneal sensitivity it is particularly useful for diabetic keratopathy, which is a typical form of corneal epithelial disorder that accompanies damage, degeneration or paralysis of corneal nerve.

### [Industrial Applicability]

The medicament of the present invention has an effect to improve decrease of corneal sensitivity and therefore, it is useful as a therapeutic agent for neurotrophic keratopathy and further, diabetic keratopathy, which is a complication of diabetes.

This application is based on a patent application No. 2015-116974 filed in Japan, the contents of which are incorporated in full herein.

## Claims

1. A therapeutic agent for neurotrophic keratopathy comprising [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof.

2. The therapeutic agent according to claim 1, wherein the neurotrophic keratopathy is neurotrophic keratopathy caused by diabetes.

3. The therapeutic agent according to claim 2, wherein the neurotrophic keratopathy caused by diabetes is diabetic keratopathy.

4. An agent for improving decrease of corneal sensitivity, comprising [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof.

5. Use of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof for the manufacture of a therapeutic agent for neurotrophic keratopathy.

6. The use according to claim 5, wherein the neurotrophic keratopathy is neurotrophic keratopathy caused by diabetes.

7. The use according to claim 6, wherein the neurotrophic keratopathy caused by diabetes is diabetic keratopathy.

8. Use of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof for the manufacture of an improving agent for decrease of corneal sensitivity.

9. A method of treating neurotrophic keratopathy, comprising administering an effective amount of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof to a subject in need of a treatment of neurotrophic keratopathy.

10. The method according to claim 9, wherein the neurotrophic keratopathy is neurotrophic keratopathy caused by diabetes.

11. The method according to claim 10, wherein the neurotrophic keratopathy caused by diabetes is diabetic keratopathy.

12. A method of improving decrease of corneal sensitivity, comprising administering an effective amount of [3-[2-[4-isopropyl-2-(4-trifluoromethyl)phenyl-5-thiazolyl]ethyl]-5-methyl-1,2-benzisoxazol-6-yl]oxyacetic acid or a pharmacologically acceptable salt thereof to a subject in need of an improvement of decrease of corneal sensitivity.
